# EUROPEAN PATENT APPLICATION

(11) **EP 4 640 243 A1**
(43) Date of publication of application: **29.10.2025**
(21) Application number: 24398008.3
(22) Date of filing: 26.04.2024
(51) Int. Cl.: A61L 2/20, B65B 55/00, C01B 13/10, F01L 9/20

(54) **SYSTEM FOR THE OZONIZATION OF CONTAINERS**

(71) Applicant: Gomes Correia, Roberto, Catia la Mar, Vargas State (VE)
(72) Inventor: Gomes Correia, Roberto, Catia la Mar, Vargas State (VE)
(74) Representative: Sousa Ferreira, Vânia

(57) **Abstract**

The invention is a system and device for the disinfection of closed containers, through the use of gaseous ozone generated on site by a novel ozone generator with high and low concentration cells, powered by an oxygen generator with its own electrical supply and comprised of a mixing device and a special connection device to the container vents, which allows the injection of ozone at very short times, with the capacity to invade all the areas and crevices within the container and the cargo, producing the disinfection and elimination of viruses, fungi, bacteria and other microorganisms from the outside of the container, for periods of time longer than the required regardless of the environmental conditions of temperature and humidity outside, ozone being a powerful viricide, which does not leave any type of waste inside the containers.

## Description

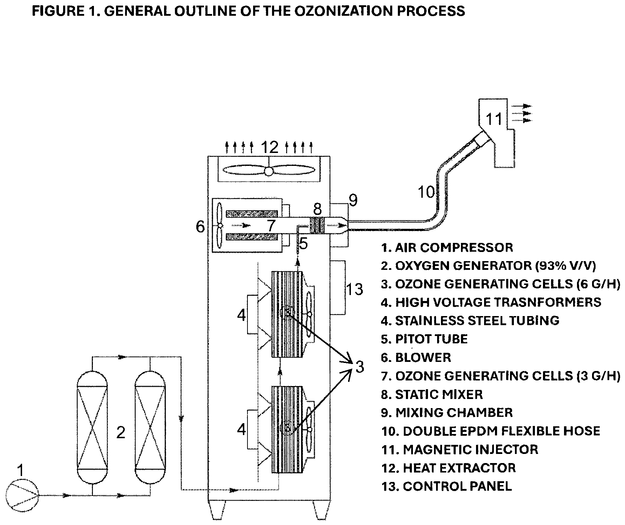

The process of generating and injecting ozone gas into the containers is comprised of a system with an air compressor, connected to a generator that in turn feeds an ozone generator, which incorporates two (2) cells in series to produce high concentration ozone in laminar flow and low concentration ozone (figure 1).

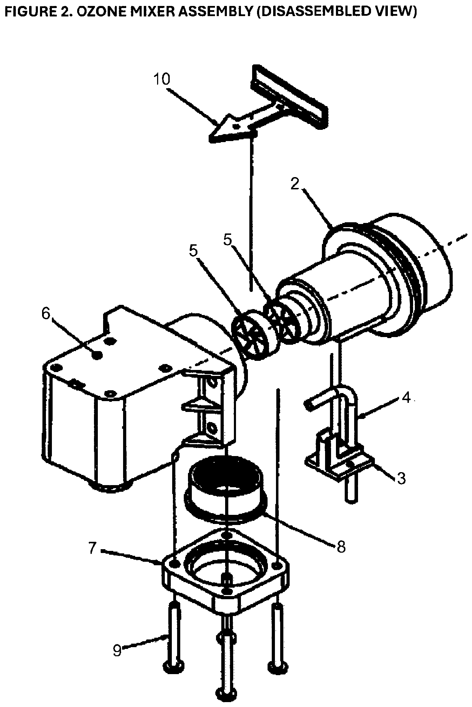

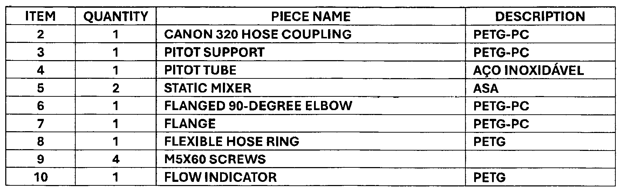

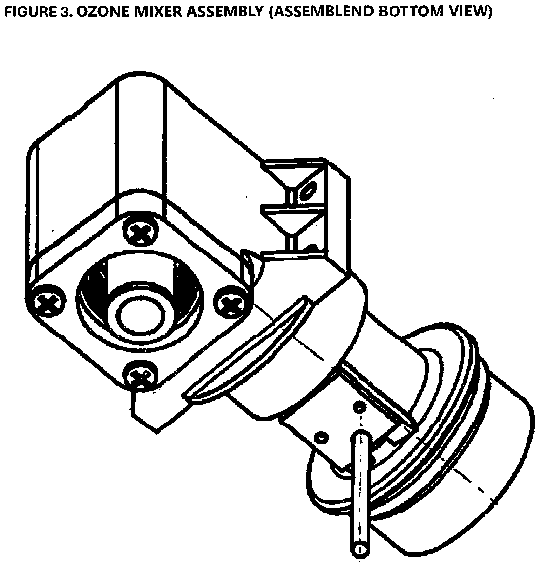

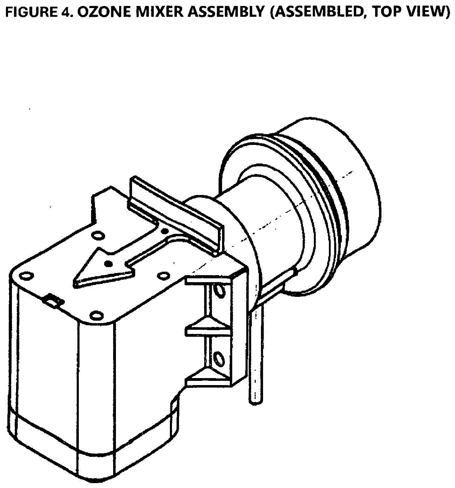

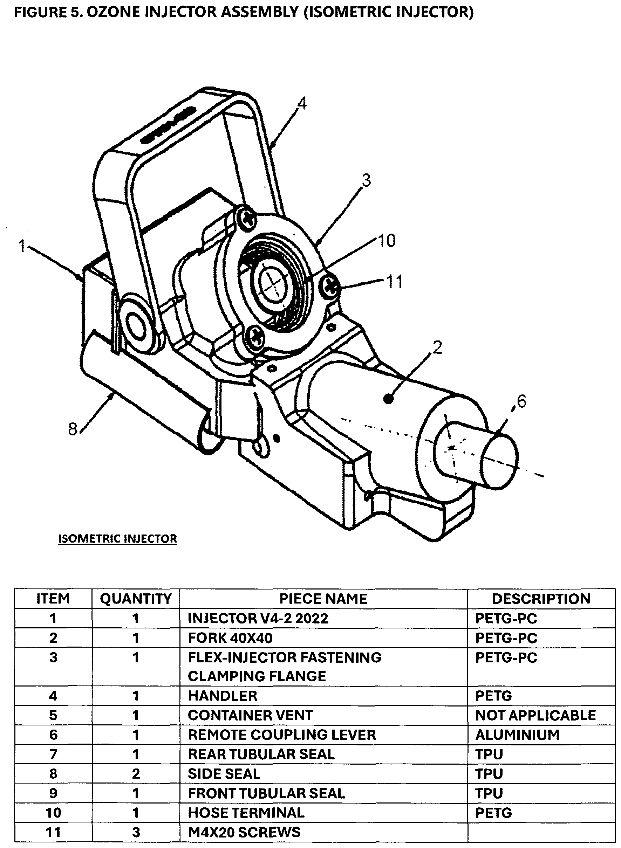

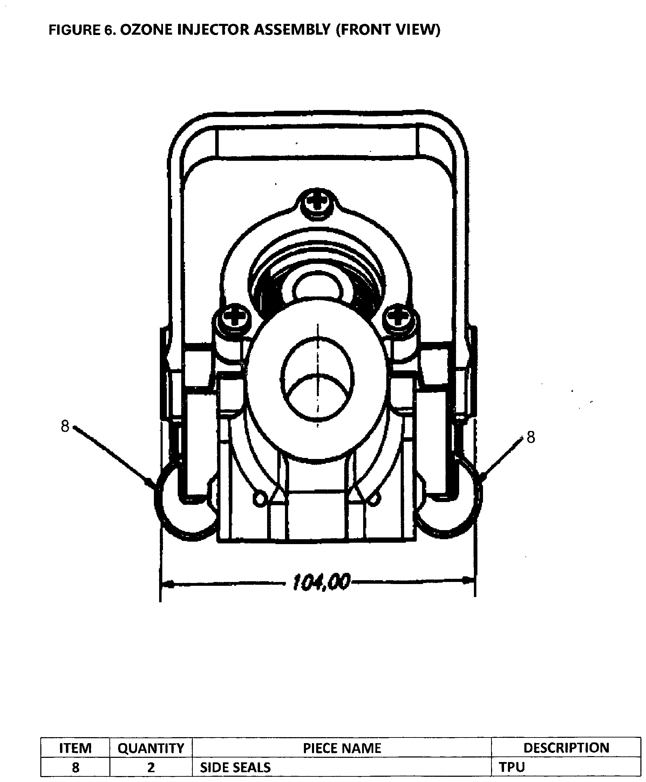

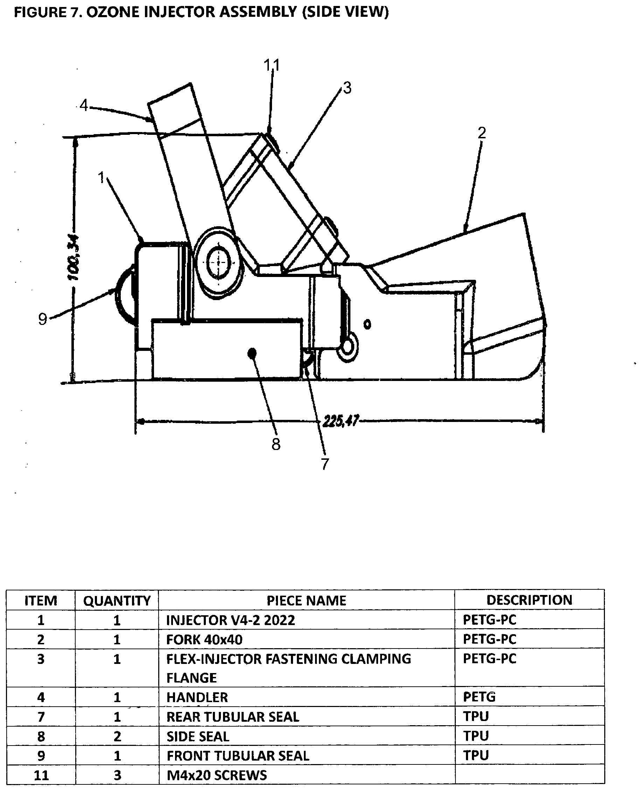

The ozone generated by the two (2) cells in series is perfectly mixed in a novel device (figures 2, 3 and 4) with oxygen and nitrogen, to subsequently be diluted in a turbulent flow through a special designed tubular element (double EPDM flexible hose) to guarantee the homogenization of both streams and have the necessary potential energy to flow through the vent and reach the ozone injector assembly (figures 5, 6 and 7) at one of the ends of the container, specifically at its vent.

The ozone injector assembly is coupled to the container vent, using a magnetic nozzle made of ozone-resistant materials (EPDM, Silicone, ABS, PEGT, TPU) that allows the entry of ozone, oxygen and nitrogen through a slightly pressurized current into the containers through the vents, at the speed necessary to being the diffusion and disinfection process of the interior of the container.

This system allows the physical phenomenon known as Fick's Law to be carried out inside the container, allowing a concentration gradient at the ends so that the ozone molecules travel in the axial axis until reaching the desired concentration (greater than 0.1 ppm) at which time the disinfection process begins throughout the receptacle.

Ozone, due to its gaseous nature, can invade all places and crevices within the container and cargo. Effectively achieving the disinfection process of exposed surfaces in less than 8 minutes.

The concentrations defined for the disinfection of viruses and bacteria and the low exposure time of ozone inside the containers can fully guarantee that there will be no type of effect on materials or electronic components (according to ISA-Class GX, for electronic instruments or equipment).

The characteristics of this system allow for an expeditious and effective disinfection process of closed containers, thanks to the innovative devices and elements that compose it which are part of the invention.

## Claims

1. A system for ozonation of the internal places of closed containers (1), which allows their disinfection, composed as follows:
a) An air compressor, which powers an oxygen generator (93% V/V)
b) Stainless steel tubing, through which oxygen flows to the ozone generating cells;
c) Two ozone generating cells (6 g/h) and two high voltage transformers.
d) Pitot tube through which ozone flows to the ozone generation cell (3g/h)
e) Blower that drives the generated ozone to a static mixer.
f) Mixing chamber (2)
g) Double EPDM flexible hose (3), through which the ozone flows to the magnetic injector assembly (4) and which is connected to the container vent.

2. A device that allows the mixing of gases, according to claim 1, comprised of a mixing chamber (2), which has a flange connection, made of PETG (Polyethylene terephthalate glycol) or PC (Polycarbonate) connected to a flex hose ring, made of PETG (Polyethylene terephthalate glycol), which is coupled to a 90 flanged elbow, made of PETG (Polyethylene terephthalate glycol), or PC (Polycarbonate), which is in turn coupled with two static mixers made of ASA (Acrylonitrile styrene acrylate) and a PITOT tube made of stainless steel with its appropriate support, which connects with hose coupling, made of polyester glycol PETG (Polyethylene terephthalate glycol) or PC (Polycarbonate).

3. A double EPDM flexible hose (3), according to claim 1, made of double EPDM (Ethylene propylene diene monomer) which connects the gas mixing assembly with the ozone injector assembly.

4. A device that allows the injection of ozone generated inside the container, according to claim 1, comprised of a magnetic injector assembly (4) with a hose terminal, made of PETG (Polyethylene terephthalate glycol), which is coupled with the flexible hose that conveys the ozone; flex-injector fastening clamping flange made of PETG (Polyethylene terephthalate glycol) or PC (polycarbonate) and attached with three M4x20 screws; 40x40 fork, made of PETG (Polyethylene terephthalate glycol) or PC (polycarbonate); remote coupling lever, made of aluminum; handler, made of PETG (Polyethylene terephthalate glycol); front tubular, rear tubular and side seals, made of TPU (thermoplastic polyurethane); V4-2 injector, made of PETG (Polyethylene terephthalate glycol) or PC (polycarbonate), ending in the magnetic nozzle (coupling) that connects to the container vent, made of ozone-resistant materials: EPDM (Ethylene propylene silicone monomer), Acrylonitrile, ABS (Butadiene styrene) PETG (Polyethylene terephthalate glycol), TPU (Thermoplastic polyurethane).
